# EUROPEAN PATENT SPECIFICATION

(11) **EP 3 007 772 B1**
(45) Date of publication and mention of the grant of the patent: **21.02.2018**
(21) Application number: 14744159.6
(22) Date of filing: 12.06.2014
(51) Int. Cl.: A61P 3/06, A61K 36/062, A61K 31/455, A61K 36/82, A61K 31/355, A61K 31/4415, A61K 31/714, A61K 31/519

(54) **COMPOSITIONS FOR USE IN THE TREATMENT OF DYSLIPIDAEMIA IN CHRONIC KIDNEY DISEASE**
COMPOSITIONS POUR LEUR UTILISATION DANS LE TRAITEMENT DE LA DYSLIPIDÉMIE DANS LA MALADIE RÉNALE CHRONIQUE
ZUSAMMENSETZUNGEN ZUR VERWENDUNG BEI DER BEHANDLUNG DER DYSLIPIDÄMIE IM CHRONISCHEN NIERENLEIDEN

(30) Priority: 14.06.2013 IT MI20130989
(43) Date of publication of application: 20.04.2016
(73) Proprietor: Difass International S.r.l., 47853 Coriano (RN) (IT)
(72) Inventor: GUASTI, Pier, Luigi, 47924 Rimini (RN) (IT)
(74) Representative: Bianchetti Bracco Minoja S.r.l.
(86) International application number: PCT/IB2014/062170
(87) International publication number: WO 2014/199333

(56) References cited:
- US-A1- 2012 171 285
- US-A1- 2012 171 311
- US-A1- 2012 172 425
- CHUN-LIN LEE ET AL: "Monascus fermentation of dioscorea for increasing the production of cholesterol-lowering agent-monacolin K and antiinflammation agent-monascin", APPLIED MICROBIOLOGY AND BIOTECHNOLOGY, vol. 72, no. 6, 28 March 2006 (2006-03-28) , pages 1254-1262, XP019441683, SPRINGER, BERLIN, DE ISSN: 1432-0614, DOI: 10.1007/S00253-006-0404-8
- CICERO A F G ET AL: "Eulipidemic effects of berberine administered alone or in combination with other natural cholesterol-lowering agents: A single-blind clinical investigation", ARZNEIMITTEL FORSCHUNG. DRUG RESEARCH, vol. 57, no. 1, 1 January 2007 (2007-01-01), pages 26-30, XP008082945, ECV EDITIO CANTOR VERLAG, AULENDORF, DE ISSN: 0004-4172
- HEBER D ET AL: "Cholesterol-lowering effects of a proprietary Chinese red-yeast-rice dietary supplement", THE AMERICAN JOURNAL OF CLINICAL NUTRITION, vol. 69, no. 2, 1 February 1999 (1999-02-01), pages 231-236, XP002253415, AMERICAN SOCIETY FOR NUTRITION, US ISSN: 0002-9165
- OSAMA GHEITH ET AL: "Efficacy and safety of Monascus purpureus Went rice in subjects with secondary hyperlipidemia", CLINICAL AND EXPERIMENTAL NEPHROLOGY, vol. 12, no. 3, 26 March 2008 (2008-03-26) , pages 189-194, XP019595441, OFFICIAL PUBLICATION OF THE JAPAN SOCIETY OF NEPHROLOGY, SPRINGER-VERLAG, TO ISSN: 1437-7799
- BABU P V A ET AL: "Green tea impedes dyslipidemia, lipid peroxidation, protein glycation and ameliorates Ca<2+>-ATPase and Na<+>/K<+>-ATPase activity in the heart of streptozotocin-diabetic rats", CHEMICO-BIOLOGICAL INTERACTIONS, vol. 162, no. 2, 25 August 2006 (2006-08-25), pages 157-164, XP027877327, ELSEVIER SCIENCE IRLAND, IR ISSN: 0009-2797 [retrieved on 2006-08-25]
- ACCINNI R ET AL: "Effects of combined dietary supplementation on oxidative and inflammatory status in dyslipidemic subjects", NMCD. NUTRITION METABOLISM AND CARDIOVASCULAR DISEASES, vol. 16, no. 2, 1 March 2006 (2006-03-01), pages 121-127, XP024989474, MILAN, IT ISSN: 0939-4753, DOI: 10.1016/J.NUMECD.2005.05.006 [retrieved on 2006-03-01]
- DATABASE MEDLINE [Online] US NATIONAL LIBRARY OF MEDICINE (NLM), BETHESDA, MD, US; 15 May 2003 (2003-05-15), MCKENNEY JAMES: "Niacin for dyslipidemia: considerations in product selection.", XP002714319, Database accession no. NLM12789870 & MCKENNEY JAMES: "Niacin for dyslipidemia: considerations in product selection.", AMERICAN JOURNAL OF HEALTH-SYSTEM PHARMACY : AJHP : OFFICIAL JOURNAL OF THE AMERICAN SOCIETY OF HEALTH-SYSTEM PHARMACISTS 15 MAY 2003, vol. 60, no. 10, 15 May 2003 (2003-05-15), pages 995-1005, ISSN: 1079-2082
- DATABASE MEDLINE [Online] US NATIONAL LIBRARY OF MEDICINE (NLM), BETHESDA, MD, US; 1994, MENENDEZ R ET AL: "Policosanol inhibits cholesterol biosynthesis and enhances low density lipoprotein processing in cultured human fibroblasts.", XP002714320, Database accession no. NLM8728831 & MENENDEZ R ET AL: "Policosanol inhibits cholesterol biosynthesis and enhances low density lipoprotein processing in cultured human fibroblasts.", BIOLOGICAL RESEARCH 1994, vol. 27, no. 3-4, 1994, pages 199-203, ISSN: 0716-9760
- ROBERTO MENÉNDEZ ET AL: "Effects of policosanol treatment on the susceptibility of low density lipoprotein (LDL) isolated from healthy volunteers to oxidative modification in vitro", BRITISH JOURNAL OF CLINICAL PHARMACOLOGY, vol. 50, no. 3, 24 September 2000 (2000-09-24), pages 255-262, XP055081904, ISSN: 0306-5251, DOI: 10.1046/j.1365-2125.2000.00250.x
- X. QIN ET AL: "Folic Acid Therapy and Cardiovascular Disease in ESRD or Advanced Chronic Kidney Disease: A Meta-Analysis", CLINICAL JOURNAL OF THE AMERICAN SOCIETY OF NEPHROLOGY, vol. 6, no. 3, 1 March 2011 (2011-03-01), pages 482-488, XP055135638, ISSN: 1555-9041, DOI: 10.2215/CJN.05310610
- TANIA PERRINJAQUET-MOCCETTI ET AL: "Food supplementation with an olive (Olea europaea L.) leaf extract reduces blood pressure in borderline hypertensive monozygotic twins", PHYTOTHERAPY RESEARCH, vol. 22, no. 9, 1 September 2008 (2008-09-01), pages 1239-1242, XP002660461, JOHN WILEY & SONS LTD. CHICHESTER, GB ISSN: 0951-418X, DOI: 10.1002/PTR.2455 [retrieved on 2008-08-27]
- NGAMUKOTE SATHAPORN ET AL: "Cholesterol-lowering activity of the major polyphenols in grape seed.", MOLECULES, vol. 16, no. 6, 2011, pages 5054-5061, XP002728824, (BASEL, SWITZERLAND) ISSN: 1420-3049
- CLAUDIA STEFANUTTI ET AL: "Combined Treatment with Dif1stat and Diet Reduce Plasma Lipid Indicators of Moderate Hypercholesterolemia More Effectively than Diet Alone: A Randomized Trial in Parallel Groups", LIPIDS, vol. 44, no. 12, 1 December 2009 (2009-12-01), pages 1141-1148, XP055136060, ISSN: 0024-4201, DOI: 10.1007/s11745-009-3368-5
- DATABASE MEDLINE [Online] US NATIONAL LIBRARY OF MEDICINE (NLM), BETHESDA, MD, US; June 2008 (2008-06), VAKNIN YIFTACH ET AL: "The potential of milk thistle (Silybum marianum L.), an Israeli native, as a source of edible sprouts rich in antioxidants.", XP002728825, Database accession no. NLM17852500 & VAKNIN YIFTACH ET AL: "The potential of milk thistle (Silybum marianum L.), an Israeli native, as a source of edible sprouts rich in antioxidants.", INTERNATIONAL JOURNAL OF FOOD SCIENCES AND NUTRITION JUN 2008, vol. 59, no. 4, June 2008 (2008-06), pages 339-346, ISSN: 0963-7486
- Dr Stefano and Laura Brunori: "Ristatin capsule", Farmacia Brunori Store , 23 May 2011 (2011-05-23), XP002728826, Retrieved from the Internet: URL:http://www.farmaciabrunori.it/en/catal ogo/ristatin_capsule-3540.html [retrieved on 2014-08-22]
- FABIO CLAUDIA STEFANUTTI: "Efficacy and Safety of Dif1stat for the Treatment of Secondary Dyslipidemia in Chronic Kidney Disease", LIFE SCIENCES, 1 January 2013 (2013-01-01), XP055136061, ISSN: 0024-3205

## Description

### Technical field of invention

The present invention is defined in claims 1-15 and it relates to compositions comprising red yeast rice dried extract, at least one straight-chain aliphatic alcohol having 14 to 36 carbon atoms, niacin, green tea dried extract, vitamin E, vitamin B6, vitamin B12, folic acid, which are useful in the treatment of dyslipidaemia in patients suffering from chronic kidney disease.

### Background to the invention

Chronic kidney disease (CKD), characterised by a progressive decline in kidney function, is a pathological condition that is constantly increasing. The main causes of CKD are the progressive aging of the population and renal complications of systemic disorders which are widespread among the population (such as arterial hypertension, diabetes mellitus II and dyslipidaemia).

Dyslipidaemia in patients suffering from chronic kidney disease represents one of the major risk factors for mortality and morbidity due to cardiovascular events, and for the progress of kidney disease.

An increased risk of cardiovascular disease is known to begin in the very first stages of CKD. The lipid profile of the majority of CKD patients is characterised by both qualitative and quantitative alterations of the circulating lipoproteins. Said profile is characterised by a reduction in HDL cholesterol (High-Density Lipoprotein) (CHDL) and in the total cholesterol (CT) and LDL cholesterol (Low-Density Lipoprotein) (CLDL) values, which may be reduced, normal or slightly increased, whereas the blood triglyceride level is often increased by low catabolism of lipoproteins rich in triglycerides (TG). The resistance of the peripheral tissue to the action of insulin, secondary hyperparathyroidism and uraemic toxins are the main factors responsible for an altered lipid profile in patients with chronic kidney disease.

According to recent NHANES (National Health and Nutrition Examination Survey) data, about 13% of the adult population of the USA suffers from chronic kidney disease (CKD) (1). A close connection has been found from a reduction in the glomerular filtration rate (GFR) and an increased risk of mortality, cardiovascular events and hospitalisation (2). Hypertension, diabetes mellitus and dyslipidaemia are well-known risk factors not only for cardiovascular disease, but also for chronic kidney disease. Measures designed to prevent or delay the onset of CKD include treatment of hypertension, blood glucose control, blocking the renin-angiotensin system and giving up smoking (3; 4). However, despite the strict application of said protocol, kidney disease is often progressive, so further measures are both desirable and necessary.

It has been postulated that dyslipidaemia may be a major potential risk factor for the progress of kidney disease, probably because it accelerates intrarenal atherosclerosis, and because of the direct toxic effect of the lipids on the kidney cells; in fact, an accumulation of lipoproteins in the renal mesangium promotes glomerulosclerosis (5). Dyslipidaemia can be corrected by statins which also have anti-inflammatory, antioxidant and antithrombotic properties, and are useful to generate additional kidney-protecting benefits (6). Animal models demonstrate that statins inhibit interstitial fibroblast proliferation and matrix synthesis, both of which factors can prevent tubulointerstitial fibrosis (7). *In vitro,* statins negatively regulate mesangial production of protein-1, M-CSF (macrophage colony-stimulating factor) and macrophage migration inhibitory factors (8; 9). Other pleiotropic properties of statins relate to their beneficial effects on the endothelial function and renal haemodynamics, and their antioxidant properties. However, the exact biological mechanism is not yet fully understood.

Although statins appear to reduce urine protein levels and slow the loss of kidney function (10; 11; 12), contradictory data have emerged from randomised controlled trials (13; 14). One study published in 2007 (15) demonstrates that treatment with 80 mg of atorvastatin a day in patients with coronary disease generated a significant increase in the estimated GFR values compared with treatment with 10 mg of atorvastatin, suggesting that the kidney-protecting effect of atorvastatin is dose-dependent. A recent review of 27 randomised clinical trials suggests that statins reduce the rate of kidney function loss by 1.22 mL/min/year more than a placebo, and also reduce the renal protein excretion rate (12). Those results are contradicted by an analysis of a sub-group of the CARE (Cholesterol and Recurrent Events) study, which concludes that the estimated decline in GFR in the group treated with pravastatin was no different from that of the group treated with the placebo (13). More recently, a meta-analysis of randomised controlled trials conducted on CKD patients demonstrated that compared with a placebo, statins reduce evident proteinuria to a modest extent, but do not modify the rate of decline of the kidney function (16). To summarise, the effect of statins on the progress of kidney disease in humans remains uncertain, but the results of the trials conducted to date suggest that in the case of patients with CHD, statins may have a therapeutic role in the progress of CKD (17). Statins are now considered as first-choice agents for the correction of dyslipidaemia, including in dyslipidaemic patients with chronic kidney failure; treatment with statins helps to reduce the cardiovascular risk factors and potentially slows the progress of kidney disease towards end-stage kidney failure.

Statins, namely drugs that inhibit endogenous cholesterol synthesis by acting on the enzyme hydroxymethylglutaryl-CoA reductase (which converts the 3-hydroxy-3-methylglutaryl-CoA molecule to mevalonic acid, a precursor of cholesterol), are generally well tolerated. However, they are not devoid of side effects, which can limit their use.

### Hepatotoxicity

On the basis of the data obtained from clinical trials and reviews of clinical reports, an increase in transaminase levels, evaluated in liver function tests (LFTs), to clinically significant levels was observed in between 0.5% and 2% of patients who take statins. They are generally dose-dependent (18; 24; 25). The liver dysfunctions induced by statins are usually rare, and accompanied by asymptomatic increases in transaminase levels or acute cholecystitis. In most cases the transaminase values return to normal when the dose of the medicament administered is reduced, but in some very rare cases, the symptoms may worsen, and kidney failure may result (18). Cholestasis and active liver disorders are contraindications to the use of statins.

### Myopathy and Rhabdomyolysis

Statins are associated with muscle disorders such as muscle weakness, cramp and myalgia, with or without elevated creatine kinase (CK) levels, a slight increase in CK levels, myositis and rhabdomyolysis (19). Myalgia is the least severe, but most frequent symptom of muscle toxicity. Rhabdomyolysis potentially associated with kidney failure is the least frequent, but also the most severe disorder. It is a syndrome deriving from damage to the sarcolemma which leads to the release into the bloodstream of substances contained in the skeletal muscle cells (myoglobin, uric acid and electrolytes). There are many causes of this disorder, including treatment with statins (20). On the basis of the results of 30 randomised clinical trials, myalgia was observed in from 0 and 32.9% of patients (as against 33.2% in the control groups). The clinical practice data demonstrate that myalgia was reported in a range of between 1% and 5% for the majority of patients treated with statins. Myositis was observed in 49 patients out of 42,323 treated with statins (0.12%), and in 44 patients out of the 41,535 controls (0.11%). Rhabdomyolysis proved to be a rare event, with only 7 cases (0.02%) in the groups treated with statins and 5 cases (0.01%) in the control groups (19). These observations were confirmed by Andrade et al. (21), who reported an 0.01% incidence of severe myopathy and rhabdomyolysis among 200,000 hospitalised patients who were new users of statins or fibrates. The development of myopathy and rhabdomyolysis is relatively uncommon for all the statins currently available (25). According to an analysis conducted by the FDA, myopathy has an incidence of 0.2%, and rhabdomyolysis of 0.01% (22).

### Other adverse effects

Other symptoms which may be observed include dyspepsia, and rarely nausea, headache or malaise. However, the causal relationship with the treatment remains unclear. In 2005 the FDA drew up a document suggesting that patients of Asian ethnicity should begin rosuvastatin treatment at the dose of 5 mg/day for the treatment of dyslipidaemia. This advice was based on a pharmacokinetics study conducted on healthy volunteers, wherein the plasma concentrations of rosuvastatin were found to be about twice as high in participants of Asian ethnicity as in Caucasians to whom the same doses of the medicament were administered. In Japan, all the approved statins present lower doses, and the maximum recommended dose is less than half the maximum dose approved for use in the USA. Moreover, in the USA, the FDA has not approved the dose of 80 mg for rosuvastatin because of the greater risk of myopathy associated with the use of this statin. In patients who suffer from serious organ dysfunctions or take medicaments liable to interact, and elderly people, statin treatment should involve the medicaments that elicit the smallest number of pharmacological interactions, such as pravastatin and rosuvastatin (the latter being more active than the former). In any event, the patients included in these categories must be closely monitored (23). Doctors should pay particular attention to evaluating signs and symptoms correlated with muscle and/or liver damage, and should investigate non-specific symptoms that may be correlated with the treatment (18).

There is still a need to find new, improved compositions for the treatment of dyslipidaemia which, in particular, are suitable for use and offer benefits to patients suffering from kidney disease.

### Summary of the invention

The present invention is defined in claims 1-15 and it relates to compositions comprising red yeast rice dried extract, at least one straight-chain aliphatic alcohol having 14 to 36 carbon atoms, niacin, green tea dried extract, vitamin E, vitamin B6, vitamin B12, folic acid for use in the treatment of dyslipidaemia in patients suffering from chronic kidney disease.

### Detailed description of the invention

It has surprisingly been found that a composition comprising red yeast rice dried extract, at least one straight-chain aliphatic alcohol having 14 to 36 carbon atoms, niacin, green tea dried extract, vitamin E, vitamin B6, vitamin B12, folic acid is useful in the treatment of dyslipidaemia in patients suffering from chronic kidney disease (CKD).

As the adverse effects of treatment with statins appear to be directly proportional to the dose of statin, a composition according to the invention containing a natural statin obtained from red yeast rice, called monacolin K, administered at lower daily doses than those used for statin-based medicaments and optionally combined with other active ingredients, provides a similarly effective treatment of dyslipidaemia, but with a much lower risk of side effects and better tolerability than medicaments based on the known statins.

Red yeast rice is the product of fermentation of rice by a fungus, *Monascus purpureus,* which produces various substances that modulate the lipid level in the blood, known as "monacolins". The monacolin most active for these purposes is "monacolin K", the content of which in red yeast rice depends on the strain of *Monascus purpureus* used and the fermentation conditions.

The red yeast rice used in the composition according to the present invention is preferably obtained under standardised fermentation conditions, with particular strains of *Monascus purpureus* selected to obtain an optimum yield of monacolin K and a specific, titrated content of said ingredient.

The monacolin K content of dried red yeast rice preferably ranges from 0.4 to 10% by weight, and more preferably from 1.5 to 5% by weight.

The composition according to the invention comprises, per daily dose, an amount of red yeast rice, preferably the dried extract, that supplies a total monacolin K intake preferably ranging from 0.5 to 40 mg, more preferably from 2 to 25 mg, most preferably from 3 to 15 mg.

According to a preferred aspect of the invention, the total monacolin K intake provided is 3 mg/day.

The composition also includes at least one straight-chain aliphatic alcohol having 14 to 36 carbon atoms, that may be present in an amount per dosage unit ranging from 0.5 to 60 mg, preferably from 1 to 20 mg, most preferably from 2 to 15 mg.

In a preferred embodiment, the composition comprises a mixture of straight-chain aliphatic alcohols having 14 to 36 carbon atoms with the following structural formula:

CH₃-(CH₂)ₙ-CH₂OH (I)

wherein n is an integer from 12 to 34.

The mixture of straight-chain aliphatic alcohols preferably contains octacosanol, a straight-chain alcohol of formula:

CH₃-(CH₂)₂₆-CH₂OH (II)

in an amount ranging from 10 to 90% by weight.

The mixture of straight-chain aliphatic alcohols can be obtained from a number of natural sources, including wheat germ wax, rice germ wax, carnauba wax (exudate of the leaves of *Copernicia prunifera*), sugar cane wax, beeswax, avocado oil, alfalfa, bamboo and apple wax.

Depending on the specific origin of the mixture of straight-chain aliphatic alcohols, the octacosanol content can preferably range from 14 to 22% by weight (beeswax policosanols), from 50 to 60% (supercritical extract of rice wax policosanols) and from 50 to 80% (sugar cane wax policosanols).

In addition to octacosanol, the mixture of straight-chain aliphatic alcohols preferably includes tetracosanol (an alcohol with 24 carbon atoms), hexacosanol (an alcohol with 26 carbon atoms), triacontanol (an alcohol with 30 carbon atoms) and dotriacontanol (an alcohol with 32 carbon atoms).

In a further preferred embodiment, the composition according to the invention therefore contains a mixture of straight-chain aliphatic alcohols having 24 to 32 carbon atoms.

The composition also includes niacin, that may be present in an amount per dosage unit ranging from 4 to 800 mg, preferably from 8 to 200 mg, most preferably from 10 to 50 mg.

In the ambit of the present invention, the term "niacin" indicates both nicotinic acid and the amide thereof, nicotinamide (otherwise known as vitamin PP or vitamin B3).

The composition also includes green tea (*Camellia sinensis*); the daily green tea intake preferably ranges from 1 mg to 1000 mg.

The composition can also include, per dosage unit, a folic acid derivative, namely pteroyl glutamic acid, and/or at least one vitamin, such as thiamine (vitamin B1), riboflavin (vitamin B2), pantothenic acid (vitamin B5).

In the ambit of the present invention, folic acid and the derivative thereof are distinguished from one another by the state of reduction of the pteridine ring, the type of monocarbon unit bonded to it, and the number of glutamic acid residues.

In the ambit of the present invention, the term "vitamin B6" indicates a complex of three compounds metabolically convertible to one another, namely pyridoxine (C₈H₁₁N0₃), pyridoxal and pyridoxamine, and their respective phosphoric esters.

In the ambit of the present invention, the term "vitamin B12" indicates a complex of substances characterised by a corrin ring containing a cobalt atom, known as cobalamins. The best-known forms thereof are hydroxocobalamin (natural) and cyanocobalamin of formula C₆₃H₈₈CoN₁₄O₁₄P, which is the commercially available form.

In the ambit of the present invention, the term "vitamin E" indicates a complex of substances generally known in the industry as tocopherols, based on α-tocopherol of chemical formula C₂₉H₅₀O₂.

The composition preferably comprises, per daily dose:
- 40 to 800 µg of folic acid and derivatives thereof; and/or
- 0.1 to 40 mg of vitamin B6; and/or
- 0.05 to 50 µg of vitamin B 12; and/or
- 8 to 64 mg of vitamin E.

More preferably, the composition comprises, per daily dose:
- 60 to 500 µg of folic acid and derivatives thereof; and/or
- 0.5 to 20 mg of vitamin B6; and/or
- 0.5 to 25 µg of vitamin B 12; and/or
- 12 to 52 mg of vitamin E.

Most preferably, the composition comprises, per daily dose:
- 100 to 400 µg of folic acid and derivatives thereof; and/or
- 0.8 to 10 mg of vitamin B6; and/or
- 0.8 to 3 µg of vitamin B 12; and/or
- 15 to 43 mg of vitamin E.

The composition can also include an omega-3 fatty acid such as eicosapentaenoic acid (EPA), also known as icosapentaenoic acid, docosahexaenoic acid (DHA), or EPA/DHA mixtures. The daily intake of omega-3 fatty acid preferably ranges from 250 mg to 1000 mg.

A further subject of the present invention is an antidyslipidaemic composition comprising dried extract of red yeast rice combined with an omega-3 fatty acid selected from eicosapentaenoic acid (EPA), docosahexaenoic acid (DHA) and EPA/DHA mixtures.

In compositions containing dried extract of red yeast rice and at least one omega-3 fatty acid, the dried extract of red yeast rice preferably provides a monacolin K intake of 5 to 10 mg/day.

According to a further aspect of the invention, the antidyslipidaemic composition comprising dried extract of red yeast rice combined with an omega-3 fatty acid selected from eicosapentaenoic acid (EPA), docosahexaenoic acid (DHA) or EPA/DHA mixtures also includes a straight-chain aliphatic alcohol having 14 to 36 carbon atoms, and niacin.

The compositions can also include anthocyanins (water-soluble pigments belonging to the flavonoid family), obtainable from various sources such as bilberry (*Vaccinium myrtillus*), karkadé (*Hibiscus sabdariffa*), chokeberry (*Aronia melanocarpa*), grapevine (*Vitis vinifera*) and blackcurrant (*Ribes nigrum*) in amounts ranging from 0.1 mg to 1000 mg per daily dose.

The compositions can also include at least one dried extract such as *Olea europaea* dried extract, *Vitis vinifera* dried extract, fenugreek dried extract, and silymarin from milk-thistle dried extract (*Silybum marianum*).

The invention can also include coenzyme Q10; the daily intake of coenzyme Q10 preferably ranges from 0.1 to 200 mg.

According to a particularly preferred aspect, the composition for use in the treatment of dyslipidaemia in patients suffering from chronic kidney disease comprises:
- Dried extract of green tea in an amount ranging from 1 mg to 1000 mg per daily dose,
- Vitamin E in an amount ranging from 8 to 64 mg per daily dose,
- Vitamin B6 in an amount ranging from 0.1 to 40 mg per daily dose,
- Vitamin B12 in an amount ranging from 0.05 to 50 µg per daily dose,
- Folic acid in an amount ranging from 40 to 800 µg.

The composition according to the invention also contains, per dosage unit, at least one excipient acceptable from the dietary standpoint, in particular a dietary excipient selected from those commonly used in the field of formulation of dietary compositions and diet supplements.

The composition according to the invention can also be formulated in solid or liquid form, depending on the nature of the various ingredients used, employing well-known preparation techniques.

The composition according to the invention can therefore be advantageously formulated in the form of oral capsules, tablets, pearls, soft capsules, soluble powder, syrup, solution, suspension or drops, food preparations, fruit purees, and milk- or yoghurt-based drinks.

The examples given below further illustrate the invention.

### Examples

### Example 1

The composition in tablet form according to the invention, prepared by known methods from commercially available powdered ingredients, comprises:

| **Ingredient** | **Amount (mg)** |
|---|---|
| Red yeast rice, dried extract, assay value 1.5% | 200 |
| Straight-chain aliphatic alcohols (octacosanol content 60%) | 10 |
| Niacin | 27 |
| Green tea, dried extract, assay value 40% | 144 |
| Vitamin E acetate 50% | 43.88 |
| Vitamin B6 (pyridoxine) | 2.44 |
| Vitamin B12 (cyanocobalamin) | 0.001 |
| Folic acid | 0.3 |

### Example 2

| **Ingredient** | **Amount (mg)** |
|---|---|
| Red yeast rice, dried extract, assay value 1.5% | 400 |
| Linear aliphatic alcohols | 2 |
| Niacin | 9 |
| Folic acid and derivatives thereof | 0.1 |
| Vitamin B6 | 1 |
| Vitamin B12 | 0.5 x 10⁻³ |
| Vitamin E | 21 |
| Magnesium stearate | 20 |

### Example 3

| **Ingredient** | **Amount (mg)** |
|---|---|
| Red yeast rice, dried extract, assay value 1.5% | 480 |
| Straight-chain aliphatic alcohols | 2.5 |
| Niacin | 10 |
| Folic acid and derivatives thereof | 0.15 |
| Vitamin B6 | 1.2 |
| Vitamin B12 | 0.6 x 10⁻³ |
| Vitamin E | 25 |
| Magnesium stearate | 20 |

### Example 4

| **Ingredient** | **Amount (mg)** |
|---|---|
| Red yeast rice, dried extract, assay value 1.5% | 600 |
| Straight-chain aliphatic alcohols | 2.8 |
| Niacin | 15 |
| Folic acid and derivatives thereof | 0.2 |
| Vitamin B6 | 1.2 |
| Vitamin B12 | 0.8 x 10⁻³ |
| Vitamin E | 29 |
| Magnesium stearate | 20 |

### Example 5

| **Ingredient** | **Amount (mg)** |
|---|---|
| Red yeast rice, dried extract, assay value 1.5% | 167 |
| Straight-chain aliphatic alcohols | 10 |
| Niacin | 28 |
| Green tea, dried extract, assay value 20% | 144 |
| Vitamin E acetate 50% | 36 |
| Vitamin B6 (pyridoxine) | 3 |
| EPA/DHA | 618 |
| Folic acid | 0.3 |

### Evaluation of antidyslipidaemic activity

The results of the two-year study described below, obtained by treating 1104 dyslipidaemic patients suffering from chronic kidney disease with one capsule a day of the product described in example 1 according to the present invention, demonstrated its properties involving control of dyslipidaemia and slowing of the progress of kidney damage in patients with mild, moderate or severe chronic kidney failure. In particular, the variations in the parameters plasma LDL cholesterol (C-LDL), plasma triglycerides (TG) and glomerular filtration rate (GFR) after 2 years' treatment with the product described in example 1 were statistically significant, similarly to the variations in the same parameters obtained by treating dyslipidaemic patients suffering from chronic kidney disease with various commercial medicaments containing statins of various types.

The benefit for the patient is the use of a product which, by exploiting the unexpected synergy of action of its nutritional ingredients, including a natural statin with a very low daily dose (3 mg/day of monacolin K was used in the study), gives similar results to those obtainable with the administration of statin-based drugs, but greatly reduces the risk of adverse side effects.

The product described in example 1 is therefore particularly useful for dyslipidaemic patients suffering from chronic kidney disease, in particular those intolerant of statins.

The compositions according to the invention can also be statin-based medicaments in all cases wherein a gradual increase in the statin dose required to reach the patient's lipid goal is inadvisable.

The compositions according to the invention can also be used as an alternative to pharmacological treatment if the patient rejects it.

Finally, in non-dyslipidaemic patients suffering from chronic kidney disease, it offers a treatment with high tolerability that slows the progress of kidney damage.

### Purpose of study

The purpose of this 24-month observational study was to evaluate the efficacy of the product, combined with a hypolipidaemic diet, on the lipid profile of patients with chronic kidney disease (stage II, III or IV of chronic kidney failure (CKF) according to the Kidney Disease Outcomes Quality Initiative (KDOQI) classification).

Its tolerability and safety were evaluated on the basis of the appearance of an elevated urine albumin content.

**Patients (Table 1)**

| **Group of 1104 patients** | **Male 65% (717)** | **Female 35% (387)** | **Age Years ± SD** | **Stage of CKD** | **GFR ml/min/ 1.73 m2** |
|---|---|---|---|---|---|
| A (180) | 61.1% (110) | 38.88% (70) | 69±10 | II° | 67±16 |
| B (744) | 69% (514) | 31% (23) | 70±13 | III° | 38±12 |
| C (180) | 51.6% (93) | 48.3% (87) | 71.8±11 | IV° | 195±6 |

1104 patients with a mean age of 70 ± 15 years were treated. 65% male and 35% female.

The patients were divided into three groups (A, B, C), according to their baseline kidney function.

Group A comprised 180 patients with an estimated glomerular filtration rate (GFR) of 67 ± 16 ml/min/m² (CKF stage II according to the KDOQI classification).

Group B comprised 744 patients with an estimated glomerular filtration rate (GFR) of 38 ± 12 ml/min/m² (CKF stage III according to the KDOQI classification).

Group C comprised 180 patients with an estimated glomerular filtration rate (GFR) of 19 ± 6 ml/min/m² (CKF stage IV according to the KDOQI classification).

### Results

**Group A (Table 2)**

| CKD stage I according to the KDOQI classification | | | | | | | | | | | | | |
|---|---|---|---|---|---|---|---|---|---|---|---|---|---|
| 180 patients with a glomerular filtration rate (GFR) calculated as 67 ± 16 ml/min/m². | | | | | | | | | | | | | |
| The percentage variation (Δ%) and the mean changes (mg/dL ± SD) in the plasma lipids, lipoprotein profile and baseline GFR after 6 (T6), 12 (T12), 18 (T8) and 24 (T24) months are reported. | | | | | | | | | | | | | |
| Months | T0 | T6 | Δ% | P | T12 | Δ% | P | T18 | Δ% | P | T24 | Δ% | P |
| TC | 251.4±41 | 212±23 | - 15.6 | n.s. | 196.2±39 | -22 | 0.001 | 186±62 | -26 | 0.001 | 172±83 | -31 | 0.001 |
| HDLC | 46±12 | 48±16 | 4.3 | n.s. | 50±21 | 8.6 | n.s. | 51±18 | 10.8 | n.s. | 52±35 | 13 | n.s. |
| TG | 152±46 | 120±75 | -21 | 0.001 | 106±45 | -30 | 0.001 | 98±61 | -35 | 0.001 | 96±40 | - 36.8 | 0.001 |
| LDLC | 175±38 | 140±47 | -20 | 0.001 | 125±52 | -28 | 0.001 | 115±62 | -34 | 0.001 | 101±51 | -42 | 0.001 |
| nonHDLC | 205±23 | 164±41 | -20 | 0.001 | 146±19 | -29 | 0.001 | 135±26 | -34 | 0.001 | 120±31 | -41 | 0.001 |
| TC/HDLC | 5.4 | 4.4 | -19 | n.s. | 3.9 | -28 | 0.001 | 3.6 | -33 | 0.001 | 3.3 | 40 | 0.001 |
| GFR | 67±16 | 67.2±14 | 0.3 | n.s. | 68±17 | 1.2 | n.s. | 68.5±16 | 2.23 | 0.001 | 68.7±12 | 2.5 | 0.001 |
| urine albumin | negative | negative | n.c. | n.c. | negative | n.c. | n.c. | negative | n.c. | n.c. | negative | n.c. | n.c. |

HDLC (HDL cholesterol), LDLC (LDL cholesterol), TC (total cholesterol), nonHDLC (non-HDL cholesterol), TG (triglycerides), n.s. = not significant, n.c. = not calculated, value p≤0.001

The variations in CT (-15.6%), CHDL (4.3%), the CT/CHDL ratio (-19%) and GFR (0.3%) were not statistically significant, while the variations in TG (-21%), CLDL (-20%) and nonCHDL (-20%) were statistically significant for P<0.001 after 6 months' treatment.

After 12 months, the reductions in CT (-22%), TG (-30%), CLDL (-28%) nonCHDL (-29%) and TC/CHDL (-28%) were statistically significant for P<0.001, whereas no statistically significant variations were observed in CHDL (8.6%) or GFR (1.2%).

After 18 months a statistically significant increase in GFR was observed (+2.23%), and the same finding was made for CT (-26%), TG (-35%), CLDL (-34%), nonCHDL (-34%) and CT/CHDL (-33%), which were statistically significant for P<0.001. The variation in CHDL (10.8%) was not significant. The reductions in CT (-31%), TG (-36.8%), CLDL (-42%), nonCHDL (-41%) and CT/CHDL (-40%) were statistically significant for P<0.001, as was the increase in GFR (+2.5%), while are no significant variations were observed in CHDL after 24 months' treatment.

**Group B (Table 3)**

| CKD stage II according to the KDOQI classification | | | | | | | | | | | | | |
|---|---|---|---|---|---|---|---|---|---|---|---|---|---|
| 744 patients with a glomerular filtration rate (GFR) calculated as 38 ± 12 ml/min/m². | | | | | | | | | | | | | |
| The percentage variation (Δ%) and the mean changes (mg/dL ± SD) in the plasma lipids, lipoprotein profile and baseline GFR after 6 (T6), 12 (T12), 18 (T8) and 24 (T24) months are reported. | | | | | | | | | | | | | |
| Months | T0 | T6 | Δ% | P | T12 | Δ% | P | T18 | Δ% | P | T24 | Δ% | P |
| TC | 269.8±65 | 249.8±73 | -7.4 | n.s. | 232±59 | -14 | n.s. | 218±46 | -19 | n.s. | 195.2±81 | -27 | 0.001 |
| HDLC | 42±19 | 42.4±22 | 0.95 | n.s. | 43±24 | 2.38 | n.s. | 43.2±31 | 2.9 | n.s. | 43.5±47 | 3.5 | n.s. |
| TG | 184±99 | 162±152 | -12 | n.s. | 147±90 | -20 | 0.001 | 131±103 | -28 | 0.001 | 125±94 | -32 | 0.001 |
| LDLC | 190±48 | 174±94 | -8.4 | n.s. | 156±56 | -18 | n.s. | 148±71 | -22 | 0.001 | 126±34 | -33 | 0.001 |
| nonHDLC | 227.8±52 | 207.4±44 | -8.9 | n.s. | 189±39 | -17 | n.s. | 174.8±63 | - 23.2 | 0.001 | 151.7±33 | - 33.4 | 0.001 |
| TC/HDLC | 6.42 | 5.89 | -8.2 | n.s. | 5.39 | -16 | n.s. | 5.04 | - 21.4 | 0.001 | 4.48 | -30 | 0.001 |
| GFR | 38±12 | 38.1±16 | 0.26 | n.s. | 38.5±17 | 1.31 | 0.001 | 38.6±16 | 1.57 | 0.001 | 38.8±11 | 2.1 | 0.001 |
| urine albumin | negative | negative | n.c. | n.c. | negative | n.c. | n.c. | negative | n.c. | n.c. | negative | n.c. | n.c. |

HDLC (HDL cholesterol), LDLC (LDL cholesterol), TC (total cholesterol), nonHDLC (non-HDL cholesterol), TG (triglycerides), n.s. = not significant, n.c. = not calculated, value p≤0.001

Patients with moderate stage III CKF (GFR 59-30 ml/min/1.73m²).

The variations in CT (-7.4%), CHDL (0.95%), TG (-12%), CLDL (-8.4%), CT/CHDL (-8.2%), non-CHDL (-8.9%) and GFR (0.26%) were not statistically significant after 6 months' treatment.

After 12 months' treatment the variations in TG (-20%) and GFR (+ 1.31 %) were statistically significant for P<0.001, while the variations in CT (-14%), CHDL (2.38%), CLDL (-18%), non-CHDL (-17%) and CT/CHDL (-16%) were not significant.

After 18 months' treatment, the variations in GFR (+1.57%), CT/CHLD (-21.4%), non-CHDL (-23.2%), CLDL (-22%) and TG (-28%) were statistically significant for P<0.001, while the variations in CT (-19%) and CHDL (+2.9%) were not significant.

After 24 months, the variation in CHDL (+3.5%) was not significant, whereas the variations in CT (-27%), TG (-32%), CLDL (-33%), nonCHDL (-33.4%), CT/HDL (-30%) and GFR (+2.1%) were statistically significant for P<0.001.

**Group C (Table 4)**

| CKD stage III according to the KDOQI classification | | | | | | | | | | | | | |
|---|---|---|---|---|---|---|---|---|---|---|---|---|---|
| 180 patients with a glomerular filtration rate (GFR) calculated as 19 ± 6 ml/min/m². | | | | | | | | | | | | | |
| The percentage variation (Δ%) and mean changes (mg/dL ± SD) in the plasma lipids, lipoprotein profile and baseline GFR after 6 (T6), 12 (T12), 18 (T8) and 24 (T24) months are reported. | | | | | | | | | | | | | |
| Months | T0 | T6 | Δ% | P | T12 | Δ% | P | T18 | Δ% | P | T24 | Δ% | P |
| TC | 285±57 | 251±29 | -12 | n.s. | 219±32 | -23 | 0.001 | 210±41 | -26 | 0.001 | 193.4±53 | -32 | 0.001 |
| HDLC | 35±19 | 31±16 | -11 | n.s. | 36±22 | 2.8 | n.s. | 38.2±29 | 9.1 | n.s. | 39.8±18 | 13 | n.s. |
| TG | 222±57 | 186±63 | -16 | n.s. | 152±41 | -31 | 0.001 | 141±59 | -36 | 0.001 | 137±39 | -38 | 0.001 |
| LDLC | 196±71 | 173±96 | - 11.7 | n.s. | 151±85 | -23 | 0.001 | 143±94 | -27 | 0.001 | 126±45 | -35 | 0.001 |
| nonHDLC | 250±33 | 220±42 | -12 | n.s. | 183±67 | -26.8 | 0.001 | 171.8±49 | - 31.8 | 0.001 | 153±56 | -38.5 | 0.001 |
| TC/HDLC | 8.14 | 8 | - 0.56 | n.s. | 6.08 | -25.2 | 0.001 | 5.49 | - 32.4 | 0.001 | 4.85 | - 40.32 | 0.001 |
| GFR | 19±6 | 191.5±5 | 0.52 | n.s. | 19.25±7 | 1.31 | 0.001 | 19.38±2 | 2 | 0.001 | 19.42±1 | 2.1 | 0.001 |
| urine albumin | negative | negative | n.c. | n.c. | negative | n.c. | n.c. | negative | n.c. | n.c. | negative | n.c. | n.c. |

HDLC (HDL cholesterol), LDLC (LDL cholesterol), TC (total cholesterol), nonHDLC (non-HDL cholesterol), TG (triglycerides), n.s. = not significant, n.c. = not calculated, value p≤0.001

Patients with severe stage IV CKF (GFR 29-15 ml/min/1.73 m²).

After 6 months' treatment, no statistically significant variations were observed in CT (-12%), CHDL (-11%), TG (-16%), CLDL (-11.7%), CT/CHDL (-0.56%), nonCHDL (-12%) or GFR (+0.52%). After 12 months, the variation in CHDL (+2.8%) was not significant, while the variations in CT (-23%), TG (-31%), CLDL (-23%), nonCHDL (-26.8%), CT/CHDL (-25.2%) and GFR (+1.31%) were statistically significant for P<0.001. After 18 months, the variation in CHDL (+9.1 %) was not significant, while the variations in CT (-26%), TG (-36%), CLDL (-27%), nonCHDL (-31.8%), CT/CHDL (-32.4%) and GFR (+2.0%) were statistically significant for P<0.001. After 24 months, the variation in CHDL (+13%) was not significant, while the variations in CT (-32%), TG (-38%), CLDL (-35%), nonCHDL (-38.5%), CT/CHDL (-40.32%) and GFR (+2.1%) were statistically significant for P<0.001.

### Conclusions

When the kidney function is most compromised, alterations in the lipid and lipoprotein profile, which become more evident, can worsen the CKF progress mechanisms, and also lead to secondary dyslipidaemia which exposes the patient to an increased cardiovascular risk as from the first stages of kidney failure.

There is therefore a need for a hypolipidaemic treatment for dyslipidaemia secondary to kidney function deficiency.

All the patients included in this study were prescribed a non-pharmacological (nutraceutical) hypolipidaemic treatment which, combined with a diet, helped to improve the plasma lipid and lipoprotein pattern. At the end of the study, none of the patients exhibited a deterioration in kidney function, expressed as the GFR value, compared with the starting values, or the appearance of albumin in the urine. None of the patients therefore needed to discontinue the hypolipidaemic treatment with Diflstat®.

Finally, the earlier hypolipidaemic treatment of patients with CKF begins, the better the effects on the lipid and lipoprotein profile, without modifying the dose of the nutraceutical.

The antiatherogenic profile of the plasma lipids and lipoproteins is therefore associated with slower progress of chronic kidney damage.

Non-pharmacological treatment, which is better tolerated than pharmacological treatment, also improves patient compliance, bearing in mind that these patients are prescribed a multidrug regimen.

The nutraceutical exhibits a hypolipidaemic action and a very low incidence of side effects.

The use of the product in the treatment of secondary dyslipidaemia in patients with CKF counteracts, with reasonable efficacy and safety, the alteration in the lipid metabolism that exacerbates the progress of chronic kidney damage.

### REFERENCES

1) Coresh J, Selvin E, Stevens LA, Manzi J, Kusck JW, Eggers P, Van Lente F, Levey AS. Prevalence of chronic kidney disease in the United States. JAMA 2007;298:2038-47.
2) Go AS, Chertow GM, Fan D, McCulloch CE, Hsu CY. Chronic kidney disease and the risks of death, cardiovascular events, and hospitalization. N Engl J Med 2004;351:1296-305.
3) Kasiske BL, O'Donnell MP, Kim Y, Keane WF. Treatment of hyperlipidemia in chronic progressive renal disease. Curr Opin Nephrol Hypertens 1993;12:602-8.
4) Keane WF. The role of lipids in renal disease: future challenges. Kidney Int 2000;57(suppl 75):527-31.
5) Kwan BC, Kronenberg F, Beddhu S, Cheung AK. Lipoproteinmetabolism and lipid management in chronic kidney disease. J Am Soc Nephrol 2007;18:1246-61.
6) Executive Summary of the Third Report of the National Cholesterol Education Program (NCEP) Expert Panel on Detection, Evaluation, and Treatment of High Blood Cholesterol in Adults (Adult Treatment Panel III). JAMA 2001;285:2486-97.
7) Hidekazu I, Takashi K, Yamashita S, Hiramatsu N, Maeshima A, Kaneko Y, Hiromura K, Ueki K, Nojima Y. Fluvastatin reduces renal fibroblast proliferation and production of type III collagen: therapeutic implications for tubulo-interstitial fibrosis. Nephron Exp Nephrol 2004;97:e115-22.
8) Kim SY, Guijarro C, O'Donnell MP, Kasiske BL, Kim Y, Keane WF. Human mesangial cell production of monocyte chemoattractant protein-1: modulation by lovastatin. Kidney Int 1995;48:363-71.
9) Miyazaki K, Isbel NM, Lan HY, Hattori M, Ito K, Bacher M, Bucala R, Atkins RC, Nikolic-Paterson DJ. Up-regulation of macrophage colony-stimulating factor and monocyte recruitment during lipid-induced glomerular injury in the exogenous hypercholesterolemic rats. Clin Exp Immunol 1997;108:318-23.
10) Tonelli M, Isles C, Craven T, Tonkin A, PfefferMA, Shepherd J, Sacks FM, Furberg C, Cobbe SM, Simes J, West M, Packard C, Curhan GC. Effect of pravastatin on rate of kidney function loss in people with or at risk for coronary artery disease. Circulation 2005;112:171-8.
11) Athyros VG, Mikhailidis DP, Papageorgiou AA, Symeonidis AN, Pehlivanidis AN, Bouloukos VI, Elisaf M. The effect of statins versus untreated dyslipidaemia on renal function in patients with coronary heart disease. A subgroup analysis of the Greek atorvastatin and coronary heart disease evaluation (GREACE) study. J Clin Pathol 2004;57:728-34.
12) Sandhu S, Wiebe N, Fried LF, Tonelli M. Statins for improving renal outcomes: a meta-analysis. J Am Soc Nephrol 2006;17:2006-16.
13) Tonelli M, Moyé L, Sacks FM, Cole T, Curhan GC. Cholesterol and Recurrent Events Trial Investigators. Effect of pravastatin on loss of renal function in people with moderate chronic renal insufficiency and cardiovascular disease. J Am Soc Nephrol 2003;14:1605-13.
14) Agarwal R. Effects of statins on renal function. Mayo Clin Proc 2007;82:1381-90.
15) Shepherd J, Kastelein J, Bittner V, Deedwania P, Breazna A, Dobson S, Wilson DJ, Zuckerman A, Wenger NK. Effect of intensive lipid lowering with atorvastatin on renal function in patients with coronary heart disease: the treating to new targets (TNT) study. Clin J Am Soc Nephrol 2007;2:1131-9.
16) Strippoli GF, Navaneethan SD, Johnson DW, Perkovic V, Pellegrini F, Nicolucci A, Craig JC. Effects of statins in patients with chronic kidney disease: meta-analysis and meta-regression of randomized controlled trials. BMJ 2008;336:645-51.
17) Huskey J, Lindenfeld J, Cook T, Targher G, Kendrick J, Kjekshus J, Pedersen T, Chonchol M. Effect of simvastatin on kidney function loss in patients with coronary heart disease. Findings from the Scandinavian Simvastatin Survival Study (4S). Atherosclerosis 205 (2009) 202-206.
18) Pasternak RC, Smith SC Jr., Bairey-Merz CN, et al. ACC/AHA/NHLBI Clinical Advisory on the Use and Safety of Statins. Stroke. 2002;33:2337-41.
19) Thompson PD, Clarkson P, Karas RH. Statin-associated myopathy. JAMA. 2003;289:1681-90.
20) Warren JD, Blumbergs PC, Thompson PD. Rhabdomyolysis: a review. Muscle Nerve. 2002; 25:332-47.
21) Andrade SE, Graham DJ, Staffa JA, Schech SD, Shatin D, La Grenade L, Goodman MJ, Platt R, Gurwitz JH, Chan KA. Health plan administrative databases can efficiently identify serious myopathy and rhabdomyolysis. J Clin Epidemiol. 2005;58:171-4.
22) Food and Drug Administration. FDA response to a citizen petition regarding Crestor (March 11, 2005). Accessed at www.fda.gov/cder/drug/infopage/rosuvastatin/crestor_CP.pdf, April 24, 2005.
23) Ballantyne CM, Corsini A, Davidson MH, Holdaas H, Jacobson TA, Leitersdorf E, März W, Reckless JP, Stein EA. Risk for myopathy with statin therapy in high-risk patients. Arch Intern Med. 2003;163:553-64.
24) Smith CC, Bernstein LI, Davis RB, Rind DM, Shmerling RH. Screening for statin-related toxicity: the yield of transaminase and creatine kinase measurements in a primary care setting. Arch Intern Med. 2003;163:688-92.
25) Gotto AM Jr. Safety and statin therapy: reconsidering the risks and benefits. Arch Intern Med. 2003;163:657-9.

## Claims

1. Composition comprising:
- red yeast rice dried extract,
- at least one straight-chain aliphatic alcohol having 14 to 36 carbon atoms,
- niacin,
- green tea dried extract,
- vitamin E,
- vitamin B6,
- vitamin B 12,
- folic acid,
for use in the treatment of dyslipidaemia in patients suffering from chronic kidney disease (CKD).

2. Composition for use according to claim 1, wherein the red yeast rice contains monacolin K in amounts ranging from 0.4 to 10% by weight.

3. Composition for use according to claim 2, wherein the amount of monacolin K ranges from 1.5 to 5% by weight.

4. Composition for use according to claims 1-3, wherein the total daily intake of monacolin K ranges from 0.5 to 40 mg, preferably from 2 to 25 mg, most preferably from 3 to 15 mg.

5. Composition for use according to claim 4, wherein the red yeast rice provides a monacolin K intake of 3 mg/day.

6. Composition for use according to claims 1-5, wherein the at least one straight-chain aliphatic alcohol having 14 to 36 carbon atoms is present in amounts ranging from 1 mg to 20 mg per dosage unit.

7. Composition for use according to claims 1-6, wherein the niacin is present in amounts ranging from 8 mg to 200 mg per dosage unit.

8. Composition for use according to claims 1-7, wherein the green tea (*Camellia sinensis*) dried extract is present in amounts ranging from 1 mg to 1000 mg per daily dose.

9. Composition for use according to claims 1-8, also containing a folic acid derivative that is pteroyl glutamic acid and/or at least one vitamin selected from the group consisting of thiamine (vitamin B1), riboflavin (vitamin B2), pantothenic acid (vitamin B5).

10. Composition for use according to claims 1-9, also containing an omega-3 fatty acid.

11. Composition for use according to claim 10, wherein the omega-3 fatty acid is selected from eicosapentaenoic acid (EPA), docosahexaenoic acid (DHA) and mixtures thereof (EPA/DHA).

12. Composition for use according to claims 1-11, also containing at least one dried extract selected from *Olea europaea* dried extract, *Vitis vinifera* dried extract, fenugreek dried extract, and silymarin dried extract from milk thistle (*Silybum marianum*).

13. Composition for use according to claims 1-12, also containing coenzyme Q10 in amounts ranging from 0.1 mg to 200 mg per daily dosage.

14. Composition for use according to claims 1-13, also containing anthocyanins in amounts ranging from 0.1 mg to 1000 mg per daily dose.

15. Composition for use according to claim 1-14, wherein:
- Dried extract of green tea is present in amounts ranging from 1 mg to 1000 mg per daily dose,
- Vitamin E is present in amounts ranging from 8 to 64 mg per daily dose,
- Vitamin B6 is present in amounts ranging from 0.1 to 40 mg per daily dose,
- Vitamin B12 is present in amounts ranging from 0.05 to 50 µg per daily dose,
- Folic acid is present in amounts ranging from 40 to 800 µg per daily dose.

## Patentansprüche

1. Zusammensetzung umfassend:
- Trockenextrakt aus Rotschimmelreis,
- wenigstens einen geradkettigen aliphatischen Alkohol mit 14 bis 36 Kohlenstoffatomen,
- Niacin,
- Trockenextrakt aus Grüntee,
- Vitamin E
- Vitamin B6,
- Vitamin B 12,
- Folsäure,
zur Verwendung in der Behandlung von Dyslipidämie in Patienten, die an chronischer Nierenerkrankung (engl. *chronic kidney disease,* CKD) leiden.

2. Zusammensetzung zur Verwendung nach Anspruch 1, wobei der Rotschimmelreis Monacolin K in Mengen von 0,4 bis 10 Gew.-% enthält.

3. Zusammensetzung zur Verwendung nach Anspruch 2, wobei die Menge an Monacolin K von 1,5 bis 5 Gew.-% reicht.

4. Zusammensetzung zur Verwendung nach den Ansprüchen 1 bis 3, wobei die tägliche Gesamtaufnahme von Monacolin K von 0,5 bis 40 mg reicht, bevorzugt von 2 bis 25 mg, besonders bevorzugt von 3 bis 15 mg.

5. Zusammensetzung zur Verwendung nach Anspruch 4, wobei der Rotschimmelreis eine Monacolin K-Aufnahme von 3 mg/Tag bereitstellt.

6. Zusammensetzung zur Verwendung nach den Ansprüchen 1 bis 5, wobei der wenigstens eine geradkettige aliphatische Alkohol mit 14 bis 36 Kohlenstoffatomen im Mengen von 1 mg bis 20 mg pro Dosiseinheit vorliegt.

7. Zusammensetzung zur Verwendung nach den Ansprüchen 1 bis 6, wobei das Niacin in Mengen von 8 mg bis 200 mg pro Dosiseinheit vorliegt.

8. Zusammensetzung zur Verwendung nach den Ansprüchen 1 bis 7, wobei der Trockenextrakt aus Grüntee (*Camelia sinensis*) in Mengen von 1 mg bis 1000 mg pro Tagesdosis vorliegt.

9. Zusammensetzung zur Verwendung nach den Ansprüchen 1 bis 8, ferner enthaltend ein Folsäurederivat, das Pteroylglutaminsäure ist, und/oder wenigstens ein Vitamin ausgewählt aus der Gruppe bestehend aus Thiamin (Vitamin B1), Riboflavin (Vitamin B2), Panthothensäure (Vitamin B5).

10. Zusammensetzung zur Verwendung nach den Ansprüchen 1 bis 9, ferner enthaltend eine Omega-3-Fettsäure.

11. Zusammensetzung zur Verwendung nach Anspruch 10, wobei die Omega-3-Fettsäure ausgewählt ist aus Eicosapentaensäure (EPA), Docosahexaensäure (DHA) und Mischungen davon (EPA/DHA).

12. Zusammensetzung zur Verwendung nach den Ansprüchen 1 bis 11, ferner enthaltend wenigstens einen Trockenextrakt ausgewählt aus Trockenextrakt aus *Olea europea,* Trockenextrakt aus *Vitis vinifera,* Trockenextrakt aus Bockshornklee und Silymarin-Trockenextrakt aus Mariendistel (*Silybum marianum*).

13. Zusammensetzung zur Verwendung nach den Ansprüchen 1 bis 12, ferner enthaltend Coenzym Q10 in Mengen im Bereich von 0,1 mg bis 200 mg pro Tagesdosis.

14. Zusammensetzung zur Verwendung nach den Ansprüchen 1 bis 13, ferner enthaltend Anthocyanine in Mengen im Bereich von 0,1 mg bis 1000 mg pro Tagesdosis.

15. Zusammensetzung zur Verwendung nach den Ansprüchen 1 bis 14, wobei:
- Trockenextrakt aus Grüntee in Mengen im Bereich von 1 mg bis 1000 mg pro Tagesdosis vorliegt,
- Vitamin E in Mengen im Bereich von 8 bis 64 mg pro Tagesdosis vorliegt,
- Vitamin B6 in Mengen im Bereich von 0,1 bis 40 mg pro Tagesdosis vorliegt,
- Vitamin B12 in Mengen im Bereich von 0,05 bis 50 µg pro Tagesdosis vorliegt,
- Folsäure in Mengen im Bereich von 40 bis 800 µg pro Tagesdosis vorliegt.

## Revendications

1. Composition comprenant:
- de l'extrait sec de levure de riz rouge,
- au moins un alcool aliphatique à chaîne droite ayant 14 à 36 atomes de carbone,
- de la niacine,
- de l'extrait sec de thé vert,
- de la vitamine E,
- de la vitamine B6,
- de la vitamine B12,
- de l'acide folique,
pour une utilisation dans le traitement de la dyslipidémie chez des patients souffrant de maladie rénale chronique (MRC).

2. Composition pour une utilisation selon la revendication 1, dans laquelle la levure de riz rouge contient de la monacoline K en des quantités allant de 0,4 à 10 % en poids.

3. Composition pour une utilisation selon la revendication 2, dans laquelle la quantité de monacoline K est comprise entre 1,5 et 5 % en poids.

4. Composition pour une utilisation selon les revendications 1 à 3, dans laquelle l'apport quotidien total de monacoline K est compris entre 0,5 et 40 mg, de préférence entre 2 et 25 mg, et de manière particulièrement préférée entre 3 et 15 mg.

5. Composition pour une utilisation selon la revendication 4, dans laquelle la levure de riz rouge fournit un apport en monacoline K de 3 mg/jour.

6. Composition pour une utilisation selon les revendications 1 à 5, dans laquelle le ou les alcools aliphatiques à chaîne droite ayant 14 à 36 atomes de carbone sont présents en des quantités allant de 1 mg à 20 mg par unité posologique.

7. Composition pour une utilisation selon les revendications 1 à 6, dans laquelle la niacine est présente dans des quantités allant de 8 mg à 200 mg par unité posologique.

8. Composition pour une utilisation selon les revendications 1 à 7, dans laquelle l'extrait sec de thé vert (*Camellia sinensis*) est présent dans des quantités allant de 1 mg à 1 000 mg par dose quotidienne.

9. Composition pour une utilisation selon les revendications 1 à 8, contenant également un dérivé d'acide folique qui est l'acide ptéroyl glutamique et/ou au moins une vitamine choisie dans le groupe constitué par la thiamine (vitamine B1), la riboflavine (vitamine B2), l'acide pantothénique (vitamine B5).

10. Composition pour une utilisation selon les revendications 1 à 9, contenant également un acide gras oméga-3.

11. Composition pour une utilisation selon la revendication 10, dans laquelle l'acide gras oméga-3 est choisi parmi l'acide eicosapentaénoïque (EPA), l'acide docosahexaénoïque (DHA) et les mélanges de ceux-ci (EPA/DHA).

12. Composition pour une utilisation selon les revendications 1 à 11, contenant également au moins un extrait sec choisi parmi un extrait sec d'*Olea europaea,* un extrait sec de *Vitis vinifera,* un extrait sec de fenugrec et un extrait sec de silymarine provenant de chardon Marie (*Silybum marianum*).

13. Composition pour une utilisation selon les revendications 1 à 12, contenant également de la coenzyme Q10 en des quantités allant de 0,1 mg à 200 mg par dose quotidienne.

14. Composition pour une utilisation selon les revendications 1 à 13, contenant également des anthocyanines en des quantités allant de 0,1 mg à 1 000 mg par dose quotidienne.

15. Composition pour une utilisation selon les revendications 1 à 14, dans laquelle :
- l'extrait sec de thé vert est présent en des quantités allant de 1 mg à 1 000 mg par dose quotidienne,
- la vitamine E est présente en des quantités allant de 8 à 64 mg par dose quotidienne,
- la vitamine B6 est présente en des quantités allant de 0,1 à 40 mg par dose quotidienne,
- la vitamine B12 est présente en des quantités allant de 0,05 à 50 µg par dose quotidienne,
- l'acide folique est présent en des quantités allant de 40 à 800 µg par dose quotidienne.
